# EUROPEAN PATENT APPLICATION

(11) **EP 4 166 089 A1**
(43) Date of publication of application: **19.04.2023**
(21) Application number: 21202674.4
(22) Date of filing: 14.10.2021
(51) Int. Cl.: A61B 8/08, A61B 8/00

(54) **IMPROVING CARDIAC ULTRASOUND IMAGING**

(71) Applicant: Koninklijke Philips N.V., 5656 AG Eindhoven (NL)
(72) Inventor: YU, Jason Richard, Eindhoven (NL); GOOßEN, André, Eindhoven (NL); WEBER, Frank Michael, Eindhoven (NL); MERAL, Faik Can, Eindhoven (NL); VIGNON, Francois Guy Gerard Marie, Eindhoven (NL); NGUYEN, Man M, Eindhoven (NL); LOSSAU, Tanja, Eindhoven (NL); GROTH, Alexandra, Eindhoven (NL); WILD, Sebastian, Eindhoven (NL); WAECHTER-STEHLE, Irina, Eindhoven (NL)
(74) Representative: Philips Intellectual Property & Standards

(57) **Abstract**

The invention provides a method of imaging the heart. An ultrasound probe is controlled to receive first reflected ultrasound echo signals from the heart transmitted using first transmit parameters and an ultrasound image of the heart is created from the first reflected ultrasound echo signals using first echo signal processing parameters. Model-based segmentation is performed of the ultrasound image to identify anatomical structures of the heart, and, for at least one anatomical structure which has areas of dropout, the segmentation is used to extrapolate from the identified anatomical structures to identify the location within the image of the areas of dropout. A next ultrasound image of the heart is obtained using second, different, echo signal processing parameters and/or second, different, transmit parameters specifically for the at least one anatomical structure, or for the areas of dropout for the at least one anatomical structure, thereby to improve the signal to noise ratio at least for the areas of dropout.

## Description

### FIELD OF THE INVENTION

The invention relates to the field of cardiac ultrasound imaging. In particular, the invention relates to improving the signal to noise ratio of cardiac ultrasound images.

### BACKGROUND OF THE INVENTION

Visibility of the lateral wall in cardiac ultrasound B-mode images is a key image-quality component in cardiac ultrasound imaging. In many cardiac ultrasound images, the lateral wall is not visible due to a low signal-to-noise ratio (SNR). There are known techniques in the image-formation process to improve the SNR, most of which involve averaging over some dimension, such as using multiple transmit pulses per scanline adjusting the receive beamforming (such as the number of elevation planes) and adjusting retrospective transmit beamforming.

However, when these techniques are applied to an ultrasound image, there is a significant increase in computational complexity which will likely result in a frame rate reduction. These drawbacks may prevent these techniques from being included as part of the default image formation mode. Thus, there is a need for an improved method for improving the SNR of cardiac ultrasound images.

### SUMMARY OF THE INVENTION

The invention is defined by the claims.

According to examples in accordance with an aspect of the invention, there is provided a method of imaging the heart, comprising:
controlling an ultrasound probe to receive first reflected ultrasound echo signals from the heart, wherein the first reflected ultrasound echo signals were transmitted using first transmit parameters;
creating an ultrasound image of the heart from the first reflected ultrasound echo signals using first echo signal processing parameters;
performing model-based segmentation of the ultrasound image to identify anatomical structures of the heart;
for at least one anatomical structure which has areas of dropout, using the segmentation to extrapolate from the identified anatomical structures to identify the location within the image of the areas of dropout; and
obtaining a next ultrasound image of the heart using second, different, echo signal processing parameters and/or second, different, transmit parameters specifically for the at least one anatomical structure, or for the areas of dropout for the at least one anatomical structure, thereby to improve the signal to noise ratio at least for the areas of dropout.

An area of dropout may be an area of an anatomical structure for which the received ultrasound signals are relatively weak. An area of dropout (i.e. a dropout region) may be a region of the ultrasound image with an SNR of less than 15dB or less than 10dB.

Often, in ultrasound images where the lateral wall is not visible (or barely visible), the lateral wall is the only region of the image where an SNR improvement is required, as the rest of the image has sufficient SNR. If the location of the lateral wall was known a priori, averaging techniques for improving the SNR (i.e. by using different signal processing parameters) could be selectively applied to the regions of the image that contain the lateral wall. This would limit the drawbacks (i.e. lowered frame rate, increased computational complexity) of using the techniques. However, before performing the scan it may be difficult to determine where in the image the lateral wall would be, as this would vary from subject to subject and would depend on the location and orientation of the ultrasound probe relative to the subject's heart.

Thus, a model-based segmentation could be used to identify anatomical structures, such as the lateral wall, in the ultrasound images of the heart. The model-based segmentation may be capable of segmenting areas of anatomical structures with low SNR (i.e. areas which are barely visible in the ultrasound image). In other words, the model-based segmentation may be able to predict the location of an area of an anatomical structure (with dropout) given the other (visible) areas of the anatomical structure.

For example, the Philips HeartModel (TM) is a model-based segmentation algorithm which can identify and label the structures of the heart in ultrasound B-mode images. Thus, an initial scan could be performed using the default image formation scheme (i.e. the first echo signal processing parameters) to create an input for the heart model. Then, the model-based segmentation using the heart model could detect the regions of the image containing the lateral wall, or if the lateral wall is not visible, could predict the expected location of the lateral wall given the other heart structures.

The location of dropout may be identified based on the output of the model-based segmentation (i.e. the location of anatomical structures) with a low SNR.

The second transmit parameters and/or the second echo signal processing parameters may be selected thereby to improve the SNR of the ultrasound image relative to the first transmit parameters and/or the first echo signal processing parameters respectively.

Typically the area of dropout may cover around 10% - 25% of the scanlines for the ultrasound image. Thus, having different transmit/receive parameters for this small area of the ultrasound image will not affect the computational resources and/or frame rate as much as it would to use different transmit/receive parameters for the who ultrasound image.

Obtaining a next ultrasound image of the heart using second, different, echo signal processing parameters and/or second, different, transmit parameters may be specifically only for the at least one anatomical structure, or only for the areas of dropout.

Obtaining a next ultrasound image of the heart may comprise controlling the ultrasound probe to receive second reflected ultrasound echo signals, wherein the second reflected ultrasound echo signals were transmitted using the second transmit parameters and, optionally, applying the second, different, echo signal processing parameters to the second reflected ultrasound echo signals.

The second, different, transmit parameters may comprise different beamforming parameters compared to the first transmit parameters, wherein the second transmit parameters include, relative to the first transmit parameters, one or more of:
an increased number of transmit pulses per scanline;
an increased transmit aperture;
an increased scanline density;
a different pulse frequency; and
a different pulse waveform.

Obtaining a next ultrasound image of the heart may comprise applying the second, different, echo signal processing parameters to the first reflected ultrasound echo signals.

The first and second echo signal processing parameters may comprise the parameters for retrospective dynamic transmit focusing. For example, they may comprise the number of transmit beams which are combined per scanline. The number of transmit beams combined may be greater for the second echo signal processing parameters than for the first.

The parameters for retrospective dynamic transmit focusing may comprise the number of so-called "multi-lines in" per transmit beam. The number of multi-lines in for the second echo signal processing parameters may be greater than the number of multi-lines in for the first echo signal processing parameters. Forming multi-lines may be a pre-requisite for retrospective dynamic transmit focusing. Increasing the number of multi-lines may be required in order to increase the number of transmit beams per scanline.

The first and second echo signal processing parameters may comprise the parameters for elevational spatial compounding. For example, they may comprise the number of elevation planes. The number of elevation planes for the second echo signal processing parameters may be greater than the number of elevation planes for the first echo signal processing parameters.

The first and second echo signal processing parameters may comprise frequency compounding parameters and the frequency compounding parameters for the second echo signal processing parameters may be chosen such that they improve the contrast to noise ratio of the areas of dropout.

The first and second echo signal processing parameters may comprise spatial compounding parameters and the spatial compounding parameters for the second echo signal processing parameters may be chosen such that they improve the contrast to noise ratio of the areas of dropout.

Identifying the location within the image of the areas of dropout may comprise using a confidence measure generated by the model-based segmentation.

At least one anatomical structure may comprise the lateral heart wall.

The invention also provides a computer program comprising computer program code which is adapted, when said program is run on a computer, to implement the afore-mentioned method of imaging the heart.

The invention also provides an ultrasound imaging system for imaging the heart, comprising:
an ultrasound probe for transmitting first ultrasound signals using first transmit parameters and receiving first reflected ultrasound echo signals from the heart;
an image creating system for creating an ultrasound image of the heart using first echo signal processing parameters;
a model-based segmentation unit for identifying anatomical structures of the heart from the ultrasound image, wherein the image-based segmentation unit is adapted to extrapolate from identified anatomical structures to identify the location within the image of areas of drop out of at least one anatomical structure; and
a controller which is adapted to selected second, different, echo signal processing parameters and/or second, different, transmit parameters specifically for the at least one anatomical structure or for the areas of drop out for the anatomical structure, thereby to improve the signal to noise ratio at least for the areas of drop out.

The controller may be adapted to control the ultrasound probe to transmit second ultrasound signals using the second, different, transmit parameters and receive second reflected ultrasound echo signals from the heart and, optionally, to apply the second, different, echo signal processing parameters to the second reflected ultrasound echo signals.

The controller may be adapted to applying the second, different, echo signal processing parameters to the first reflected ultrasound echo signals.

The image-based segmentation unit may be adapted to extrapolate from identified anatomical structures to identify the location within the image of areas of drop out by using a confidence measure generated by the model-based segmentation.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Fig. 1: illustrates the general operation of an exemplary ultrasound system;
- Fig. 2: shows an illustration of ultrasound images of a heart; and
- Fig. 3: shows the output of a model-based segmentation on an ultrasound image of the heart.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method of imaging the heart. An ultrasound probe is controlled to receive first reflected ultrasound echo signals from the heart transmitted using first transmit parameters and an ultrasound image of the heart is created from the first reflected ultrasound echo signals using first echo signal processing parameters. Model-based segmentation is performed of the ultrasound image to identify anatomical structures of the heart, and, for at least one anatomical structure which has areas of dropout, the segmentation is used to extrapolate from the identified anatomical structures to identify the location within the image of the areas of dropout. A next ultrasound image of the heart is obtained using second, different, echo signal processing parameters and/or second, different, transmit parameters specifically for the at least one anatomical structure, or for the areas of dropout for the at least one anatomical structure, thereby to improve the signal to noise ratio at least for the areas of dropout.

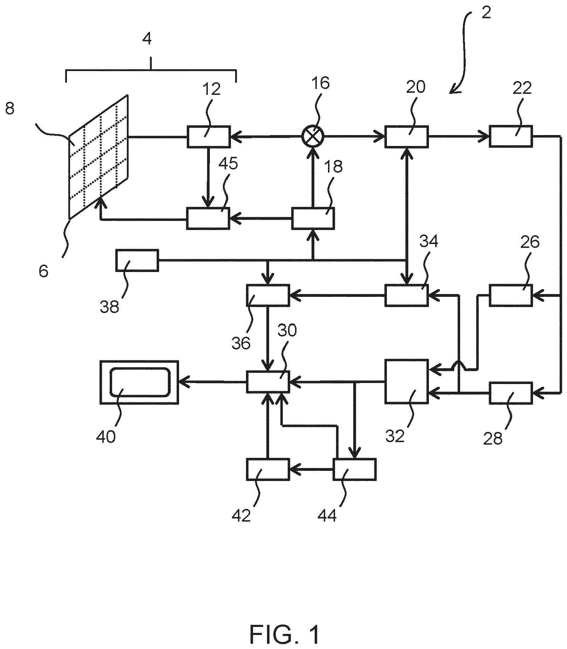

The general operation of an exemplary ultrasound system will first be described, with reference to Fig. 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The aim of the invention is to boost the signal to noise ratio (SNR) in the areas of the ultrasound image where the lateral wall is expected to appear. By using a heart model to predict the expected location of the lateral wall, SNR-boosting techniques can be selectively applied to limit the negative consequences of these techniques on frame rate and computational complexity.

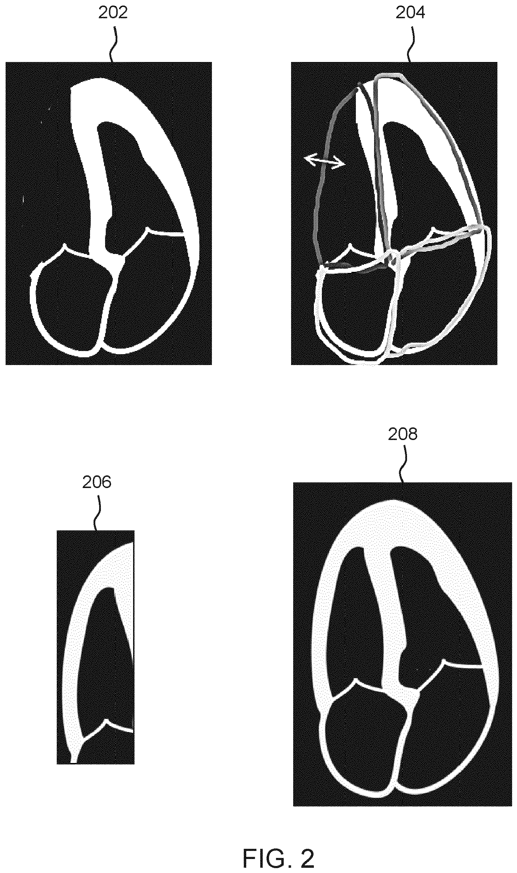

Fig. 2 shows an illustration of ultrasound images of a heart, and is used to explain the processing steps for improving the signal to noise ratio. A first B-mode ultrasound image 202 is acquired using default image formation techniques and parameters. These are first transmit parameters and first echo signal processing parameters.

In general, the first image 202 is obtained using first transmit parameters and first receive parameters (i.e. first echo signal processing parameters). Transmit parameters are parameters which affect the transmit pulses of the ultrasound probe/transducer. Receive parameters are parameters affecting the processing of the received echo signals.

A model-based segmentation (MBS) is applied to the first image 202 (e.g. using a fast heart model) in order to identify anatomical structures of the heart. The identified anatomical structures can be seen in the segmented ultrasound image 204. As can be seen in the segmented image 204, MBS is capable of identifying where regions of anatomical areas should be even if the particular area is not visible due to dropout because the ultrasound signal is too weak.

Thus, the image of the anatomical structure has areas of dropout as represented by image portion 206, and the segmentation is used to extrapolate from the identified anatomical structures in the image 204 to identify the location within the image of the areas of dropout.

For example, the region of the first image 202 that either contains the lateral wall (if the lateral wall is visible), or is expected to contain the lateral wall (if the lateral wall is not visible) is identified based on the segmentation. This can be seen in top left region of the segmented image 204 where the MBS has identified the lateral wall even when it wasn't shown in the first image 202.

MBS is known as a robust technique to apply to ultrasound images even when there is missing or noisy data. It is thus capable of predicting anatomical regions, such as the lateral wall, based on a trained constellation model. Applying MBS with a small number of mesh points and iterations allows a fast adaptation to the image with sufficient accuracy.

This allows for, for example, identifying the lateral wall region in one frame and dynamically adapting the imaging parameters (i.e. transmit and/or receive parameters) for the next frame of an ultrasound sequence. A fast model may be used to identify regions that are often subject to poor visibility or dropout.

The next step involves obtaining a second ultrasound image 208 which has a better SNR for lateral wall than the first image 202 for the dropout region 206.

This second ultrasound image may be obtained by using second, different, echo signal processing parameters and/or by using second, different, transmit parameters specifically for the at least one anatomical structure, or for the areas of dropout 206 for the at least one anatomical structure.

A first embodiment for obtaining a second image 208 involves the re-transmission of a new scanline pulse sequence for the dropout region 206. In practice, when performing cardiac ultrasound imaging, the dropout region 206 will likely be the lateral wall.

For the image lines that contain the lateral wall (i.e. corresponding to the dropout region 206), instead of transmitting one pulse at this location and acquiring one set of channel data at these locations, it is proposed to transmit N pulses and obtain N sets of channel data. For example, the number of pulses per line may be increased from one to two or three.

The transmit pulses could also be sent at different frequencies and/or with different waveforms. These changes may help to de-correlate acoustic multipath noise and thus improve the SNR for the second image 208.

Additionally, if the default image settings (i.e. first transmit parameters) are not using the largest transmit aperture (corresponding to thinnest, most focused, transmit beam), the transmit aperture can be increased for the scanlines around the dropout region 206. For example, the line density may be increased from 1 degree to 0.25 degrees.

For each of the scanline locations where multiple datasets (e.g. N transmit pulses) were acquired, the datasets which were acquired for the same location may be averaged. If the receive beamforming is based on retrospective transmit beamforming the averaging may need to be performed prior to retrospective transmit beamforming.

Retrospective transmit beamforming can be used to form a synthetically focused ultrasound image using standard (scanned and focused) ultrasound transmissions. In general, retrospective transmit beamforming is a synthetic focusing technique that uses standard, scanned-beam transmit data, dynamic receive focusing, and a combination of time-aligned data from multiple transmit beams to form images.

Beamforming could also be based multi-line beamforming. Multi-line beamforming is based on obtaining more than one scanline (i.e. multi-lines in) for a single transmit beam in order to increase the frame rate. The number of multi-lines in per transmit beam is based on the width of the transmit beam (i.e. the wider the transmit beam, the more lines which can be obtained). Multi-line beamforming may be a pre-requisite for retrospective transmit beamforming. Retrospective transmit beamforming is a known method for increasing the SNR of an ultrasound image. The number of multi-lines may be a parameter of retrospective transmit beamforming. Thus, the first and second echo signal processing parameters may comprise parameters for retrospective transmit beamforming, wherein said parameters may include the number of obtained multi-lines in and/or the number of transmit beams which are combined.

The number of multi-lines in is for example adapted prior to adapting the number of transmit beams combined in retrospective transmit beamforming.

However, the averaging of transmit pulses could be done either before or after receive multi-line formation. Generally, some form of averaging is performed over one or more of the dimensions along which several acquisitions were obtained. The averaging could be coherent and/or incoherent.

The rest of the image formation process (as discussed above) for the second image 208 may be done based on conventional processes (e.g. identical to the image formation process of the first image 202). This is possible as the changes in transmit parameters for the dropout region 206 should have improved the SNR for the lateral wall region compared to the first image 202.

In general, the first embodiment can be summarized as: identifying dropout regions 206 from a first image 202, adapting the transmit parameters (e.g. via optimization) by, for example, using known SNR improving techniques and obtaining a subsequent second image 208 where the dropout regions 206 are obtained with the adapted (second) transmit parameters. By constantly applying the MBS to each ultrasound frame, it is possible to refine a region of interest or to track it upon subject or transducer movement.

The first embodiment described above involves re-transmitting a custom pulse sequence after running MBS (e.g. the Philips Heart model) on a first ultrasound image 202. While this approach is theoretically possible, implementing the scheme may require a substantial amount of software and/or field programmable gate array (FPGA) code changes and rework. Thus, in some instances, it may be preferable not to adapt the transmit parameters.

A second embodiment is proposed that does not involve re-transmission. Instead of acquiring new echo signal data, the channel data that was used to create the first image 202 is reprocessed differently (i.e. with different receive parameters) after the MBS identifies the dropout region 206. Various SNR-boosting techniques are proposed below.

An exemplary SNR-boosting technique for the receive parameters involves, in the dropout region 206, is, when using a retrospective dynamic transmit focusing approach (i.e. a version of retrospective transmit beamforming), increasing the number of simultaneous receive beams per transmit beam (also referred to as multi-lines) formed (up to a certain point which is dependent on the width of the transmit beam), which then allows for increasing the number of transmit beams which are combined per scanline. Retrospective dynamic transmit focusing averages multiple transmit beams for a single location in order to increase the signal to noise ratio. When multiple transmit beams are combined to generate each scanline, and each transmit beam has a different noise pattern, the noise sums incoherently while the signal sums coherently, thus improving the signal-to-noise ratio.

Generally, the parameters of retrospective dynamic transmit focusing can be adapted to increase the SNR of the dropout region 206. The parameters of retrospective dynamic transmit focusing may include the number of multi-lines in and/or the number of transmit beams which are combined (e.g. averaged).

Another exemplary SNR-boosting technique for the receive parameters involves, in the dropout region 206, forming and averaging many closely spaced elevation planes used in an elevational spatial compounding approach. The averaging can be done coherently, in the beamsummed RF domain, or incoherently after log compression, in which case it is usually referred to as "elevation compounding". The number of elevation planes can only be increased up to a certain point before diminishing returns are realized as the elevation planes must all fit within the transmit elevation beam.

Generally, the parameters of elevational spatial compounding can be adapted to increase the SNR of the dropout region 206.

Adapting the amount of frequency compounding and/or performing additional spatial compounding may also be used to improve the contrast to noise ratio for the dropout region 206. However, improving the contrast to noise ratio by changing the frequency compounding and/or performing additional spatial compounding may be at the expense of resolution.

Frequency compounding is commonly used to reduce the speckle variance in ultrasound images in order to enhance contrast resolution by averaging two or more uncorrelated sub-band images (after receive beamforming). In other words, two or more different band-pass filters are applied to the received echo and the results are averaged.

Similarly, spatial compound involves obtaining echo data from several different angles and combines them (e.g. by averaging) to produce a single ultrasound image.

Increasing the number of transmit beams combined in retrospective dynamic transmit focusing and forming closely spaced elevation planes could be used together or separately.

The first embodiment (changing transmit parameters) could be combined with the second embodiment (changing receive parameters) such that a new second image 208 is obtained with different receive and transmit parameters, where the change in both receive and transmit parameters is for boosting the SNR of the dropout region 206 relative to the first image 202.

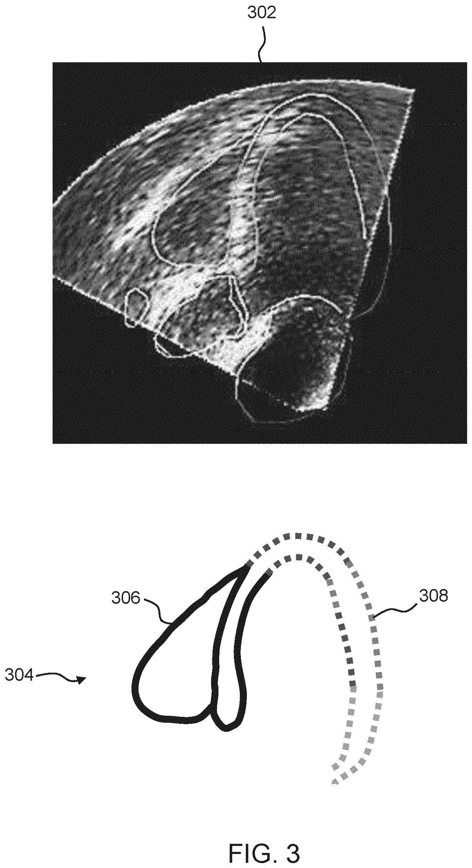

With regard to the localization of dropout regions, it is proposed to include a built-in confidence measure of the MBS in order to identify regions. Fig. 3 shows the output of a model-based segmentation on an ultrasound image of the heart 302. An MBS has been applied to the ultrasound image 302 and the results have been overlaid onto the ultrasound image 302. For illustrative purposes, a section 304 of the MBS output has been illustrated below the ultrasound image 302.

For regions 306 with high confidence (thick black lines), no beamforming parameters may need to be modified, whereas, in regions 308 of low confidence (dotted lines) beamforming may need to be adapted to achieve a higher SNR for the dropout region.

It may be advantageous to gradually steer the (computational) effort put into re-transmitting/re-computing. This ideally balances the drop in frame rate against the useful investment in SNR improvement. For example, the number N of pulses could be determined by the "weakness" of the image features and/or a determination of the SNR for the dropout region.

The skilled person would be readily capable of developing a controller for carrying out any herein described method. Thus, each step of a flow chart may represent a different action performed by a controller, and may be performed by a respective module of the processing controller.

The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method of imaging the heart, comprising:
controlling an ultrasound probe to receive first reflected ultrasound echo signals from the heart, wherein the first reflected ultrasound echo signals were transmitted using first transmit parameters;
creating an ultrasound image (202) of the heart from the first reflected ultrasound echo signals using first echo signal processing parameters;
performing model-based segmentation of the ultrasound image (202) to identify anatomical structures of the heart;
for at least one anatomical structure which has areas of dropout (206), using the segmentation to extrapolate from the identified anatomical structures to identify the location within the image of the areas of dropout (206); and
obtaining a next ultrasound image (208) of the heart using second, different, echo signal processing parameters and/or second, different, transmit parameters specifically for the at least one anatomical structure, or for the areas of dropout (206) for the at least one anatomical structure, thereby to improve the signal to noise ratio at least for the areas of dropout (206).

2. The method of claim 1, wherein obtaining a next ultrasound image (208) of the heart comprises controlling the ultrasound probe to receive second reflected ultrasound echo signals, wherein the second reflected ultrasound echo signals were transmitted using the second transmit parameters and, optionally, applying the second, different, echo signal processing parameters to the second reflected ultrasound echo signals.

3. The method of claim 2, wherein the second, different, transmit parameters comprise different beamforming parameters compared to the first transmit parameters, wherein the second transmit parameters include, relative to the first transmit parameters, one or more of:
an increased number of transmit pulses per scanline;
an increased transmit aperture;
an increased scanline density;
a different pulse frequency; and
a different pulse waveform.

4. The method of claims 1 to 3, wherein obtaining a next ultrasound image (208) of the heart comprises applying the second, different, echo signal processing parameters to the first reflected ultrasound echo signals.

5. The method of claim 4, wherein the first and second echo signal processing parameters comprise the parameters for retrospective dynamic transmit focusing including, for example, the number of transmit beams which are combined per scanline.

6. The method of claim 5, wherein the parameters for retrospective dynamic transmit focusing include the number of multi-lines in per transmit beam.

7. The method of any one of claims 4 to 6, wherein the first and second echo signal processing parameters comprise the parameters for elevational spatial compounding including, for example, the number of elevation planes.

8. The method of any one of claims 4 to 7, wherein the first and second echo signal processing parameters comprise frequency compounding parameters and/or spatial compounding parameters, wherein the frequency compounding parameters and/or spatial compounding parameters for the second echo signal processing parameters are chosen such that they improve the contrast to noise ratio of the areas of dropout (206).

9. The method of any one of claims 1 to 8, wherein identifying the location within the image of the areas of dropout (206) comprises using a confidence measure generated by the model-based segmentation.

10. The method of any one of claims 1 to 9, wherein the at least one anatomical structure comprises the lateral heart wall.

11. A computer program comprising computer program code means which is adapted, when said program is run on a computer, to implement the method of any one of clams 1 to 10.

12. An ultrasound imaging system for imaging the heart, comprising:
an ultrasound probe for transmitting first ultrasound signals using first transmit parameters and receiving first reflected ultrasound echo signals from the heart;
an image creating system for creating an ultrasound image (202) of the heart using first echo signal processing parameters;
a model-based segmentation unit for identifying anatomical structures of the heart from the ultrasound image, wherein the image-based segmentation unit is adapted to extrapolate from identified anatomical structures to identify the location within the image of areas of drop out (206) of at least one anatomical structure; and
a controller which is adapted to select second, different, echo signal processing parameters and/or second, different, transmit parameters specifically for the at least one anatomical structure or for the areas of drop out (206) for the anatomical structure, thereby to improve the signal to noise ratio at least for the areas of drop out (206).

13. The system of claim 12, wherein the controller is adapted to control the ultrasound probe to transmit second ultrasound signals using the second, different, transmit parameters and receive second reflected ultrasound echo signals from the heart and, optionally, to apply the second, different, echo signal processing parameters to the second reflected ultrasound echo signals.

14. The system of claim 12 or 13, wherein the controller is adapted to apply the second, different, echo signal processing parameters to the first reflected ultrasound echo signals.

15. The system of any one of claims 12 to 14, wherein the image-based segmentation unit is adapted to identify the location within the image of areas of drop out (206) by using a confidence measure generated by the model-based segmentation.
